(19) Europäisches Patentamt
European Patent Office
Office européen des brevets

(11) **EP 2 301 516 B1**

(12) **EUROPÄISCHE PATENTSCHRIFT**

(45) Veröffentlichungstag und Bekanntmachung des Hinweises auf die Patenterteilung:
**28.11.2018 Patentblatt 2018/48**

(51) Int Cl.:
*A61K 8/04* *(2006.01)*     *A61K 8/25* *(2006.01)*
*A61K 8/26* *(2006.01)*     *A61K 8/37* *(2006.01)*
*A61K 8/39* *(2006.01)*     *A61Q 15/00* *(2006.01)*

(21) Anmeldenummer: **10009068.7**

(22) Anmeldetag: **01.09.2010**

(54) **Aerosolzubereitungen mit stabilisierten partikulären Stoffen**

Aerosol compositions with stabilised particulate materials

Préparations d'aérosol dotées de matières particulaires stabilisées

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **28.09.2009 DE 102009043004**

(43) Veröffentlichungstag der Anmeldung:
**30.03.2011 Patentblatt 2011/13**

(73) Patentinhaber: **Beiersdorf AG**
**20253 Hamburg (DE)**

(72) Erfinder:
• **Biel, Stefan**
**21077 Hamburg (DE)**
• **Miertsch, Heike**
**22459 Hamburg (DE)**
• **Maurer, Peter**
**24536 Neumünster (DE)**
• **Heins, Sabine**
**25462 Rellingen (DE)**

(74) Vertreter: **Hartmann, Jost**
**Beiersdorf AG**
**Unnastrasse 48**
**20253 Hamburg (DE)**

(56) Entgegenhaltungen:
**EP-A1- 2 014 273**     **EP-A1- 2 090 284**
**EP-A1- 2 186 544**     **WO-A2-2010/112460**
**FR-A1- 2 859 907**

EP 2 301 516 B1

**Beschreibung**

Die Erfindung umfasst Aerosolzubereitungen umfassend ein oder mehrere pulverförmige Stoffe, mindestens ein Suspendiermittel, gewählt aus der Gruppe der Schichtsilikate, und ein Aktivierungssystem.

Aerosole, insbesondere Antitranspirantaerosole, sind i.d.R. wasserfreie Suspensionen, in denen ein oder mehrere Wirkstoffe, wie beispielsweise Antitranspirantien, in Pulverform vorliegen. Damit das Pulver in der Aerosoldose nicht zu schnell sedimentiert oder die Ventile verstopft, werden der Formulierung Suspendierhilfen zugesetzt. Als Suspendierhilfen werden zumeist Schichtsilikate gewählt.

Schichtsilikate sind polymere kristalline Natriumdisilikate, die als multifunktionelle Gerüststoffe alternativ zu Pentanatriumtriphosphat oder in Ergänzung zu Zeolith-Builder-Systemen vornehmlich in Waschmittel-Formulierungen eingesetzt werden.

Es ist bekannt, dass Schichtsilikate als Ionenaustauscher die Härtebildner des Wassers (Ca- und Mg-Ionen) binden und als Alkaliträger, Puffer und Korrosionsschutzmittel fungieren können. Bekannte Schichtsilikate sind Tonmineralien, wie Montmorrillonit, Nontronit, Hectorit, Saponit, Sauconit, Beidellit, Allevardit, Illit, Halloysit, Attapulgit und/oder Sepiolit ebenso wie Disteardimonium Hectorit. Hectorite sind $M_{0,3}^+(Mg_{2,7}Li_{0,3})[Si_4O_{10}(OH)_2]$, $M^+$ meist = $Na^+$, zu den Smektiten gehörendes, dem Montmorillonit ähnliches, monoklines Tonmineral.

Schichtsilikate werden in kosmetischen Formulierungen vor allem als Suspendierhilfen eingesetzt, d.h. in Suspensionen können sie das Sedimentieren von dispergierten Partikeln verhindern bzw. zumindest verzögern. In Emulsionen können sie auch zur Verdickung beitragen. Die dazu verwendeten und bekannten Schichtsilikate müssen laut Hersteller zur Entfaltung ihrer vollen Wirksamkeit mit polaren Zusätzen und hohen Scherkräften aktiviert werden (z.B. Produktinformationen zu Tixogel ® VP-V (Quaternium-90 Bentonite) der Fa. Rockwood Additives Ltd. oder zu Bentone® 38 (organisches Derivat eines Magnesium-Schichtsilikates (Hectorit)) der Fa. Rheox Inc.

Diese Aktivierung der Schichtsilikate, d.h. Überführung in eine quellbare Form, erfolgt indem man die Schichtsilikate mit einer polaren Flüssigkeit und hohen Scherkräften behandelt. Für wasserfreie Antitranspirant (AT)-Aerosole werden dafür als polare Flüssigkeiten z.B. Propylencarbonat, Ethanol, Dipropylenglykol eingesetzt.

DE 2442314 A1 beschreibt Puderdeodorantien mit einem Antitranspirantwirkstoff und einem Suspendiermittel in einem Treibmittel, wobei für die verminderte Fleckenbildung ein Emollientiensystem aus alkoxylierten höheren Fettalkoholen, alkoxylierten niederen Alkohol und niederem aliphatischen Alkoholester einer höheren Fettsäure gewählt werden. Als weiterer Bestandteil wird u.a. Dipropylenglykol erwähnt.

Ebenso wird in der DE 2608123 die Kombination von Suspendiermitteln und nichtflüchtige Lösemitteln erwähnt. Als eines von vielen Lösemitteln wird Dipropylenglykol (DPG) zitiert.

DE 3015450 A1 beschreibt Antitranspirantmittel als Aerosole, die neben dem Antitranspirantwirkstoff Ester der Milch-, Wein-, Adipin- und/oder Citronensäure umfassen. Die Ester sollen zur einer verbesserten AT-wirksamkeit beitragen indem sie die Feuchtigkeitspermeabilität erhöhen. Die Ester führen auch zur Verringerung der Verstopfung der Aerosolventile, da sie wasserzurückhaltend wirken und der pudrige AT-wirkstoff somit nicht verklumpt.

DE 102008022434 beschreibt kosmetischen Formulierungen mit dem Zusatz an Triethylcitrat. Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG).

DE 102007031452 beschreibt Zusammensetzungen, die sich zur Vorbeugung und Behandlung von trockener Haut eignen und eine Depot-Feuchtigkeitswirkung aufweisen. In einer Ausführungsform können die Mittel mindestens einen Deodorant-Wirkstoff enthalten. Dieser kann aus antimikrobiellen Wirkstoffen ausgewählt sein. Weiterhin wird erwähnt, einen Wirkstoff einzusetzen, der die für die Schweißzersetzung verantwortlichen Enzyme, insbesondere die Arylsulfatase, β-Glucuronidase, Aminoacylase, Esterasen, Lipasen und/oder Lipoxigenase, hemmt. Als Wirkstoffe werden u.a. z. B. Trialkylcitronensäureester, insbesondere Triethylcitrat, oder Zinkglycinat genannt. Als weitere Deodorant-Wirkstoffe sind Geruchsabsorber und desodorierend wirkende Ionenaustauscher in der DE 102007031452 benannt. Silikate dienen als Geruchsabsorber, die auch gleichzeitig die rheologischen Eigenschaften der erfindungsgemäßen Zusammensetzung vorteilhaft unterstützen. Zu den genannten Silikaten zählen vor allem Schichtsilikate, wie Montmorillonit, Kaolinit, Ilit, Beidellit, Nontronit, Saponit, Hectorit, Bentonit, Smectit und Talkum.

DE 10237054 beschreibt kosmetische und dermatologische Antitranspirant-Zubereitung mit tonmineralhaltigem Verdickersystemen.

Das Dokument EP 2014273 A1 offenbart Antitranspirant-Aerosole, welche Aluminium Chlorohydrat als pulverförmige Stoffe, ein Schichtsilikat und Propylenglykol enthalten. Die offenbarten Zubereitungen enthalten kein Triethylcitrat.

Auch aus EP 2090284 A1 sind Antitranspirante-Aerosole bekannt, welche pulverförmige Antitranspirantwirkstoffe und Schichtsilikate enthalten. Jedoch konnte auch diese Schrift keinen Hinweis auf die vorliegende Erfindung geben.

Darüber hinaus kennt der Stand der Technik die Dokumente FR 2859907 A1 und WO 2010112460 A2, welche Aerosole mit Aluminium Chlorohydrat, einem Schichtsilikat und Triethylcitrat offenbaren. Da kein Dokument Aktivierungssysteme mit Dipropylenglykol offenbart werden, konnte kein Hinweis auf die vorliegende Erfindung entnommen werden.

Im Antitranspirant (AT) - Bereich werden Schichtsilikate vor allem in wasserfreien Suspensionen eingesetzt, die als Stift oder Aerosol angeboten werden. Insbesondere ist deren Einsatz in AT-Aerosolen relevant, bei denen die Sedimentation von Partikeln in einem Öl-Treibgasgemisch verzögert werden muss.
Die Kombination mit Treibgas kann jedoch dazu führen, dass bereits aktivierte Schichtsilikat-Öl-Gemische ihre Struktur verlieren, d.h. das Pulver zu schnell sedimentiert.

Nachteilig bei den bekannten Aerosolen ist auch, dass die Suspendiermittel, wie Schichtsilikate, häufig durch Formelbestandteile, wie beispielsweise Parfumöle oder auch durch die Treibgase selber, in der Suspendierwirkung beeinträchtig werden.

Wünschenswert ist es daher Zubereitungen zur Verfügung zu stellen, die die Nachteile des Standes der Technik nicht aufweisen. Insbesondere ist es wünschenswert Antitranspirant-Aerosol Formulierungen bereit zu stellen, die trotz Zugabe polare Stoffe, wie Parfumöle oder Zugabe der Treibgase, das Absetzverhalten der partikulären Inhaltsstoffe nicht beeinflussen.

Als polare Gruppen bezeichnet man in der Chemie solche funktionellen Gruppen, deren charakteristische Elektronenverteilungen dem Molekül ein beträchtliches elektrisches Dipolmoment erteilen. Solche Gruppen bedingen die Affinität zu anderen polaren chemischen Verbindungen oder z. B. bei Tensiden zu polaren Grenzflächen, insbesondere zu Wasser. Sie sind daher auch für den hydrophilen Charakter einer Substanz verantwortlich. Im Sinne der Erfindung werden als polare Stoffe solche Substanzen verstanden, die polare funktionelle Gruppen enthalten und somit dem Stoff einen hydrophilen Charakter geben. Für Substanzgemische wie beispielsweise Parfümöle gilt die Polarität der Einzelsubstanzen, das heißt ein Parfüm kann sowohl polare als auch unpolare Substanzen enthalten. Ein Zusatz von polaren Stoffen oder Substanzgemischen, die polare Substanzen enthalten führt somit zu einer Beeinflussung der Wirkweise der Suspendierhilfe.

Die verwendeten Treibgase in AT-Aerosolen sind vorzugsweise unpolar. Vorzugsweise werden Kohlenwasserstoffe wie Gemische aus Propan, Butan und Isobutan verwendet. Durch das Treibgas wird die wasserfreie Suspension mit dem Schichtsilikat als Suspendierhilfe verdünnt, so dass bei unzureichender Aktivierung des Schichtsilikates dieser Verdünnungseffekt zu einer Strukturschwächung und somit zu einem nicht akzeptablen Absetzverhalten der Partikel führen kann.

Es sind also zwei verschieden Störfaktoren zu berücksichtigen: polare Zusätze stören die Aktivierung der Wirkstofflösung, Treibgase können das Gesamtsystem zerstören. Beide Störungen lassen sich erfindungsgemäß vermeiden bzw. vermindern.

Die Erfindung umfasst Aerosolzubereitungen umfassend ein oder mehrere pulverförmige Stoffe, mindestens ein Suspendiermittel, gewählt aus der Gruppe der Schichtsilikaten, und ein Aktivierungssystem aus Triethylcitrat (TEC) und Dipropylenglykol (DPG) im Verhältnis von 10:1 bis 1:10

Unterstützend kann der Zusatz an Wasser für das Aktivierungssystem sein. Vorteilhaft wird daher während der Aktivierung Wasser zur Aerosolzubereitung dazu gegeben.

Unter pulverförmigem oder partikulärem Stoff werden Zubereitungsbestandteile verstanden, die in einer Form zerteilter, zumeist trockener fester Stoffe vorliegen. Pulver werden durch Zerkleinern, d. h. Zerreiben oder Zerstoßen in der Reibschale (Pulverisieren), Mahlen in Mühlen oder als Folge von Zerstäubungstrocknungen oder Gefriertrocknungen erhalten. Eine besonders feine Zerteilung nennt man oft Atomisierung oder Mikronisierung. Die pulverförmigen Stoffe sind als solche in der Zubereitung enthalten oder liegen suspendiert vor. Insbesondere sind pulverförmige Stoffe pulverförmige oder partikuläre Wirk- und/oder Füllstoffe. Bevorzugte pulverförmige (partikuläre) Inhaltsstoffe sind Antitranspirantwirkstoffe, wie beispielsweise Aluminiumchlorohydrate.

Erfindungsgemäß ist zudem die Verwendung eines Aktivierungssystems aus Triethylcitrat (TEC) und Dipropylenglykol (DPG) im Verhältnis von 10:1 bis 1:10, gegebenenfalls zuzüglich Wasser, zur Aktivierung von Schichtsilikaten. Insbesondere sind Dipropylenglycol und Triethylcitrat im Verhältnis von 10:1 bis 1:10 als Aktivierungssystem für Schichtsilikate in Antitranspirant-Aerosolen zu verwenden.

Diese Systeme funktionieren besonders gut in Treibgas enthaltenen Systemen und führen nicht zu dem im Stand der Technik zu beobachteten unvorteilhaften Sedimentieren von partikulären Inhaltsstoffen, wie Antitranspirantwirkstoffen.

Nach der Aktivierung können den erfindungsgemäßen Zubereitungen weitere polare Komponenten, wie z. B. Parfümöle, zugegeben werden, ohne den Strukturaufbau der Suspendierhilfe zu zerstören. Ein ausreichendes Absetzverhalten bleibt gewährleistet.

Triethylcitrat, beispielsweise erhältlich als Citroflex® der Firma Vertellus, ist als Weichmacher in Kosmetika bekannt.
In der Lebensmittelindustrie wird Triethylcitrat als künstlicher Trägerstoff und Stabilisator für Eiklarpulver eingesetzt. In kosmetischen Formulierungen findet Triethylcitrat Anwendung als Deowirkstoff, wie in DE202006015778 U1 beschrieben. In dieser Funktion, als Deo-wirker, ist Triethylcitrat für die erfindungsgemäßen AT-Aerosole nicht relevant, da andere Antitranspirantwirkstoffe enthalten sind, die es vornehmlich zu stabilisieren gilt.

Ein wichtiger Baustein der erfindungsgemäßen Zubereitungen ist das Aktivierungssystem, der sogenannte Aktivator. Diesem kommt die Aufgabe zu, das eingesetzte Schichtsilikat, wie hydrophobiertes Tonmineral, zu delamellieren, was auch als Aktivierung bezeichnet wird. Üblicherweise werden hierzu kleine, polare Moleküle wie Propylenglycolcarbonat und Ethanol eingesetzt, die sich unter mechanischem Energieeintrag zwischen die Schichten der Tonminerallamellen schieben und somit den gewünschten Vorgang durch elektrostatische Wechselwirkung mit diesen ermöglichen. Darüber hinaus bilden sie Wasserstoffbrückenbindungen zu den delamellierten Tonmineralplättchen aus und sorgen durch diese Brückenfunktion - in einer Doppelfunktion als quasi Klammer und Scharnier für den Zusammenhalt der entstehenden spielkartenhausähnlichen Struktur.

Die beschriebenen Vorgänge auf mikroskopischer Ebene bewirken eine Verdickung der flüssigen Matrix und spiegeln sich in einer makroskopisch leicht beobachtbaren Viskositätserhöhung des Systems wieder. Typischerweise zeigen diese Systeme eine stark ausgeprägte Thixotropie.

Ein weiterer erfindungsgemäßer Teilaspekt besteht in einem besonderen Gewichtsverhältnis von Tonmineralverdicker A zu Aktivator C. Die Effizienz und rheologische Charakteristik der Viskositätserhöhung erfindungsgemäßiger Zubereitungen durch tonmineralische Verdicker A ist in entscheidendem Maße von auf das jeweilige System optimierten Abmischungverhältnissen und Herstellbedingungen abhängig. So kann ein nicht-optimiertes Gewichtsverhältnis von A zu C zu ungünstigen rheologischen Eigenschaften führen. Hierbei kann die Menge an A im Gewichtsverhältnis zu C sowohl zu niedrig als auch zu hoch sein, was sich in beiden Fällen in einer unzureichenden Verdickungsleistung und somit schneller Phasentrennung des Füllguts niederschlägt, was es erfindungsgemäß zu vermeiden gilt. Das für erfindungsgemäße Zubereitungen bevorzugte Gewichtsverhältnis von scherempfindlichem Schichtsilikaten A zum Aktivatorsystem C liegt im Bereich von 15:1 bis 1:1, vorzugsweise 10:1 bis 2:1.

Antitranspirant-Aerosole sind bekanntermaßen wasserfreie Systeme, um beispielsweise Korrosion metallischer Packungen zu vermeiden.

Die erfindungsgemäßen Zubereitungen sind prinzipiell ebenfalls wasserfrei. Wasserfrei heißt in diesem Zusammenhang, dass die Menge an zugesetztem Wasser in den Zubereitungen vorzugsweise 0,01 bis 2,0 Gew.%, bevorzugt 0,05 bis 1,75 Gew.%, besonders bevorzugt 0,5 bis 1,5 Gew.-%, bezogen auf das Gesamtgewicht der Zubereitung, beträgt.

Unter zugesetztem Wasser ist diejenige Menge an Wasser zu verstehen, die in reiner Form im Herstellungsprozess zugegeben wird. Dieses Wasser dient, wie zuvor ausgeführt, als Bestandteil des Aktivierungssystems der Aktivierung des eingesetzten Schichtsilikates und wird herstellungsbedingt durch dieses gebunden bzw. absorbiert bzw. adsorbiert. Durch dieses zugesetzte aber gebundene Wasser unterscheidet sich die erfindungsgemäße Zubereitung von bekannten "wasserfreien" Zubereitungen.

Wasser, welches darüber hinaus in den erfindungsgemäßen Rohstoffen standardmäßig enthalten ist, ist nicht als zugesetztes Wasser zu betrachten, da es unter normalen Anwendungsbedingungen der beanspruchten Zubereitungen vom Verbraucher ebenfalls nicht sensorisch oder auf sonst eine Weise wahrnehmbar ist.

Wasserfreie Zubereitung bedeutet erfindungsgemäß, dass neben dem zugesetztem Wasser lediglich zusätzliches Wasser bis zu einem Anteil von maximal 5 Gew.%, bezogen auf die Gesamtmasse der Zubereitung, enthalten sein kann. Bevorzugt bedeutet wasserfrei ein Anteil von maximal 2 Gew.%, insbesondere 0 Gew.%, bezogen auf die Gesamtmasse der Zubereitung.

Somit sind die Zubereitungen für verbrauchertypische Anwendungssituationen im Sinne einer technologischen Zuordnung als wasserfrei zu bezeichnen, obwohl sie Wasser enthalten.

Es ist bekannt, dass Dipropylenglykol und Triethylcitrat als Lösemittel in Parfüms eingesetzt werden können. So können bestimmte Riechsubstanzen besser gelöst werden.

Eine Verwendung als Aktivatorsystem für Schichtsilikate dieser Verbindungen ist nicht bekannt.

Triethylcitrat kann als alleinige polare Substanz als Aktivierungssystem oder mit anderen polaren Substanzen eingesetzt werden. Bei der anspruchsgemäßen Kombination von Triethylcitrat und Dipropylenglykol ist ein Verhältnis von 10:1 bis 1:10, vorzugsweise 5:1 bis 1:5 zu wählen. Die Kombination beider Substanzen gewährleistet, dass Bestandteile aus Parfümölen keinen Einfluss mehr auf die Strukturausbildung und somit auch auf das Absetzverhalten haben.

Die vorteilhafte Aktivierung von Schichtsilikaten mittels der erfindungsgemäßen Aktivierungssysteme zeigt sich insbesondere in nachfolgenden Vergleich des Absetzverhaltens partikulärer Stoffe im Öl-Treibgas-Gemisch.

Die Bestimmung des Absetzverhaltens wird wie folgt durchgeführt.

- Abfüllung der Wirkstofflösung mit dem Treibgas in so genannte Sicherheitsschaugläser (z. B. erhältlich von der Firma Pamasol)
- Nach Aufschütteln der Partikel wird alle 15 Sekunden an der Skalierung der Sicherheitsschaugläser (ml) der Verlauf der Phasengrenze zwischen flüssiger Phase und Partikel enthaltender Phase abgelesen

- Berechnung des Dispersionsanteils D in Abhängigkeit von der Zeit t

$$D(t) = \text{Anteil der Partikel enthaltenen Phase / Gesamtvolumen [in \%]}$$

- Berechnung des Sedimentationskoeffizienten S nach 15 s (entscheidend ist die Anfangsgeschwindigkeit innerhalb der ersten 15 s)

$$v(t) = (100-D)/t$$

Es wurden folgende Vergleichszubereitungen hinsichtlich der Absetzgeschwindigkeit von Aluminiumchlorohydrat untersucht.

Es wurde folgende Basisrezeptur verwendet (Tabelle 1):

| Tabelle 1 | Rohstoff | Menge in % (bezogen auf das Gesamtgewicht der Suspension, ohne Treibgas) |
|---|---|---|
| Phase A | Schichtsilikat (Disteardimoniumhektorit) | Siehe Tabelle 2 |
| | Aktivatoren | Siehe Tabelle 2 |
| | Cyclomethicone | Ad. 100 |
| Phase B | Aluminiumchlorhydrat | 35% |
| | Parfüm | 6,25% |
| | Octyldodecanol | 1% |
| | Dimethicone | 3% |

Die Komponenten der Phase A werden zusammen eingewogen und dann mit einem Ultraturrax bei 6000 U/min für 5min homogenisiert. Anschließend werden die Komponenten der Phase B dazugegeben und mit einem Magnetrührer oder Blattrührer homogen vermischt. Die Formulierung wird mit einem Treibgasgemisch aus Propan, Butan, Isobutan (Druckstufe 2,7 bar) abgefüllt. Das Verhältnis Wirkstofflösung zu Treibgas ist 15:85 Gew. %.

Aufgrund der oben angeführten Versuchsbeschreibung ergaben sich folgende in der Tabelle 2 dargestellten Werte für die Absetzgeschwindigkeit:

Bei den sich absetzenden Partikeln handelt es sich bei der gewählten Basisformulierung um Aluminiumchlorhydrat, welches als weißes Pulver in den Glasaerosolen gut sichtbar ist.

Tabelle 2

| Nr. | Anteil Schichtsilikat | Anteil DPG | Anteil TEC | Anteil Wasser | AbsetzGeschwindigkeit nach 15s |
|---|---|---|---|---|---|
| 1 | 4 | | | | >5 |
| 2 | 4 | | 0,5 | | 2,2 |
| 3 | 4 | | 1,5 | | 2,0 |
| 4 | 4 | | 2,5 | | 1,6 |
| 5 | 3,5 | 0,65 | 0,65 | | 1,7 |
| 6 | 3,5 | 0,65 | 0,65 | | 1,7 |
| 7 | 3,5 | 0,65 | 0,65 | | 1,3 |
| 8 | 3,5 | 0,65 | 0,65 | | 1,3 |
| 9 | 3,5 | 0,87 | 0,43 | | 1,7 |
| 10 | 3,5 | 0,43 | 0,87 | | 2,2 |
| 15 | 4 | 2,0 | | | >5 |

Der Verzicht auf ein Aktivierungsmittel bewirkt ein zu schnelles Absinken der Partikel, die Absetzgeschwindigkeit nach 15s ist oberhalb von 5 (Versuch 1). Das gleiche gilt für die alleinige Verwendung von Dipropylenglykol (Versuch 15).

Bei Verwendung von Triethylcitrat und Dipropylenglykol konnte jedoch die Absetzgeschwindigkeit deutlich reduziert werden. Je kleiner die Absetzgeschwindigkeit desto besser ist die Wirkung der Aktivierungsmittel. Dabei ist der Einfluss des nachträglich zugesetzten Parfüms nur gering (Versuche 5 bis 8).

Aufgrund der Zugabe der erfindungsgemäßen Aktivierungssystems ist es erstmals möglich Aerosolzubereitungen mit zu suspendierenden Partikeln stabil herzustellen und zu lagern. Das Aktivierungssystem Triethylcitrat und Dipropylenglykol wird anspruchsgemäß im Verhältnis von 10:1 bis 1:10, vorzugsweise 5:1 bis 1:5, besonders bevorzugt 3:1 bis 1:2 eingesetzt.

Die Aktivierungsmittel, als Gemisch aus mehreren (TEC, DPG, Wasser) werden in einer Gesamtkonzentration bezogen auf die Gesamtmasse der Zubereitung bzw.

Wirkstoffmischung ohne Treibgas im Bereich von 0,1 Gew.% bis 5 Gew.%, vorzugsweise von 0,5% bis 3%, zugesetzt. Das Verhältnis von Schichtsilikat zu Aktivierungsmittel beträgt 15:1 bis 1:1, vorzugsweise 10:1 bis 2:1.

Mit der neuen Aktivierung sind die Aerosolzubereitungen nun unabhängig von weiteren Zusätzen. Zweck des Schichtsilikates ist es, dass Absinken aller im System befindlichen Partikel zu verzögern. Das sind hauptsächlich Aluminiumchlorohydrate und andere Füllstoffe. Das Schichtsilikat selbst als Füllstoff ist zu vernachlässigen.

Als pulverförmige Wirkstoffe sind die in der Kosmetik bekannten Wirkstoffe erfindungsgemäß einsetzbar. Bevorzugt handelt es sich um Antitranspirantwirkstoffe.

Als Antitranspirantwirkstoff lassen sich vorteilhaft saure Aluminium- und/oder Aluminium/Zirkoniumsalze einarbeiten. Diese können aktiviert sein. Hierbei beziehen sich die beschriebenen Konzentrationsbereiche auf die so genannten Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Antitranspirantwirkstoffe im Sinne der vorliegenden Anmeldung sind insbesondere zu wählen aus

 1. Klassische AT-Wirker, insbesondere Metallsalze, besonders Aluminiumsalze vorzugsweise:

- Aluminum Chlorohydrate (ACH) und activated Aluminum Chlorohydrate (ACH' oder AACH)
- Aluminium Sesquichlorohydrate (ASCH), Aluminum Dichlorohydrate (ADCH)
- Aluminium-Zirkonium-Komplexe, optional umfassend Glycine (AZG), Aluminum zirconium tri-, tetra-, penta-, octa-chlorohydrate, Aluminum zirconium tri-, tetra-, penta-, octachorohydrex GLY

 ferner:

- Alumiumchoride, -sulfate
- Alaune (z.B. Ammonium Alum, Potassium Alum)
- Sodium aluminum chorhydroxy lactate und/oder

 2. Neuartige AT-Wirker, die Aluminiumfrei sind, wie insbesondere

- Anticholinergika, vorzugsweise: Glycopiperolatverbindungen, wie sie in der WO 2007141289 A1 beschrieben sind und/oder Glycopyrolat,
- Aquaporin-Inhibitoren
- Ionen-Kanal-Hemmer, vorzugsweise NKCC1-Hemmer

Die nachfolgende Auflistung vorteilhaft einzusetzender Antitranspirant-Wirker soll in keiner Weise einschränkend sein:

Aluminium-Salze (der empirischen Summenformel $[Al_2(OH)_mCl_n]$, wobei m+n=6):

- Aluminiumchlorhydrat $[Al_2(OH)_5Cl]$ x $H_2O$

 Standard Al-Komplexe: Locron L, Locron LIC, Locron LIF (Clariant), Chlorhydrol (Reheis), ACH-303 (Summit), Aloxicoll L (Giulini).
 Aktivierte Al-Komplexe: Reach 501 (Reheis), Aloxicoll 51L

- Aluminiumsesquichlorhydrat $[Al_2(OH)_{4,5}Cl_{1,5}]$ x $H_2O$

 Standard Al-Komplexe: Aloxicoll 31L (Giulini), Westchlor 186 (Westwood Chemicals)

Aktivierte Al-Komplexe: Reach 301 (Reheis)

- Aluminiumdichlorhydrat $[Al_2(OH)_4Cl_2]$ x $H_2O$

Aluminium-Zirkonium-Salze:

- Aluminium/Zirkonium Trichlorhydrex Glycin $[Al_4Zr(OH)_{13}Cl_3]$ x $H_2O$ x Gly
  Standard Al/Zr-Komplexe: Rezal 33GC (Reheis), AZG-7164 (Summit)
- Aluminium/Zirkonium Tetrachlorhydrex Glycin (GLY) $[Al_4Zr(OH)_{12}Cl_4]$ x $H_2O$ x Gly
  Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
- Aluminium/Zirkonium Pentachlorhydrex Glycin $[Al_8Zr(OH)_{23}Cl_5]$ x $H_2O$ x Gly
  Standard Al/Zr-Komplexe: Rezal 67 (Reheis), Zirkonal L540, Zirkonal L530 PG (Giulini), Westchlor ZR 80B (Westwood Chemicals)
- Aluminium/Zirkonium Octachlorhydrex Glycin $[Al_8Zr(OH)_{20}Cl_8]$ x $H_2O$ x Gly: Westchlor ZR 82B
- Reach AZP - 908 SUF activated Aluminum Zirkonium Tetrachlorohydrex Gl
- Reach AZZ - 902 SUF activated Aluminum Zirkonium Trichlorohydrex Glyc

Ebenso vorteilhaft können aber auch Glycin-freie Aluminium/Zirkonium-Salze eingesetzt werden. Bei den pulverförmigen Antitranspirantstoffen kann es sich um gemahlene oder hohle Partikel handeln.

Die Antitranspirant-Wirkstoffe aus den zuvor geschilderten Gruppe der adstringierende AT-Mittel, den klassischen AT-Mittel werden in den erfindungsgemäßen Formulierungen in einer Menge von 1 bis 40 Gew.%, vorzugsweise von 3 bis 15 Gew. %, bezogen auf die Gesamtmasse der Zubereitung, d.h. inklusive ggf. vorhandener Treibgase, eingesetzt. Bei Einsatz von ca. 35 Gew.% AACH in der Wirkstofflösung (ohne Treibgas) und einem Abfüllverhältnis von etwa 15:85 (Wirkstofflösung zu Treibgas) wird ein Anteil von etwa 5,25 Gew. % AACH im Endprodukt vorhanden sein.

AT-mittel aus der Gruppen der Anticholinergika, wie beispielsweise 4-[(2-Cyclopentyl-2-hydroxyphenylacetyl)oxy]-1,1-dimethyl-piperidiniumsalze, insbesondere das 4-[(2-Cyclopentyl-2-hydroxyphenylacetyl)oxy]-1,1-dimethyl-piperidiniumbromid können zu einem Anteil von bevorzugt 0,05 bis 1,0 Gew.%, vorzugsweise 0,1%-0,7%, insbesondere 0,3%-0,5 Gew.%, bezogen auf die Gesamtmasse der Zubereitung zugesetzt werden.

Zusätzlich ist selbstverständlich möglich weitere Antitranspirant Wirkstoffe und/oder Deodorantien zu zusetzen.

Als weitere pulverförmige Stoffe können Füllstoffe enthalten sein. Als Füllstoffe sind partikuläre Rohstoffe zu verstehen, die sich gegenüber den restlichen Formulierungsbestandteilen inert verhalten. Sie tragen im Wesentlichen zum Hautgefühl bei, können aber auch Aussehen und Struktur der Formulierung beeinflussen. Einfache, neutrale Füllstoffe sind vorzugsweise Talkum und Kaolin, aber auch Polysaccharide wie Stärken und Cellulosen und deren Derivate sind geeignet.

Die Suspendierhilfen gewählt aus der Gruppe der Schichtsilikate, z. B. Disteardimonium Hectorit, werden bevorzugt zu einem Anteil von 1 bis 10 Gew,%, insbesondere im Bereich von 2 bis 5 Gew.%, bezogen auf die Gesamtmasse der Zubereitung bzw. Wirkstoffmischung ohne Treibgas zugegeben.

Vorteilhaft sind beispielsweise die in der DE 4009347 beschriebenen Schichtsilikate, von denen insbesondere Montmorillonit, Kaolinit, Ilit, Beidellit, Nontronit, Saponit, Hectorit, Bentonit, Smectit bevorzugt sind.

Die erfindungsgemäßen Zubereitungen werden vorzugsweise dargereicht aus Standard Aerosol -Packmitteln. Insbesondere sind sie für die Versprühung durch Standard AT-Ventile und -Sprühköpfe geeignet, da sie Verstopfungsnachteile vermeiden helfen. Vorteil der erfindungsgemäßen Zubereitungen ist somit, dass auf aufwändige Spezial-Packmittel wie beispielsweise Puderventile verzichtet werden kann. Derartige Ventile sind aufgrund ihrer besonderen Konstruktion bzw. Geometrie weniger empfindlich für ein Verstopfen durch größere Partikel. Nachteilig an der Verwendung derartiger Puderventile ist aber, dass die besondere Form i.d.R. auch den Einsatz von adaptierten Sprühköpfen erfordert. Dies bedingt zusätzliche Kosten sowie eine Diversifikation des Packmittelsortiments und damit zusätzlichen logistischen Aufwand.

Diese Nachteile werden erfindungsgemäß vermieden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, UV-Filter, Antioxidantien, Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Nachfolgende Beispiele illustrieren die erfindungsgemäßen Zubereitungen. Die darin angeführten Anteile sind auf die Gesamtmasse der Zubereitung bzw. Wirkstofflösung bezogen.

**Beispiele:**

|  | Bsp. 1 | Bsp. 2 | Bsp. 3 | Bsp. 4 |
|---|---|---|---|---|
| Disteardimoniumhektorit | 0,60 | 0,52 | 0,60 | 0,52 |
| Aktivatoren | 0,37 | 0,20 | 0,30 | 0,23 |
| Cyclomethicone | 7,23 | 7,48 | 6,90 | 7,40 |
| Aluminiumchlorhydrat | 5,25 | 5,25 | 5,00 | 5,00 |
| Parfüm | 0,95 | 0,95 | 1,00 | 0,95 |
| Octyldodecanol | 0,15 | 0,15 |  |  |
| Dimethicone | 0,45 | 0,45 | 0,60 | 0,45 |
| Cellulose |  |  | 0,60 | 0,45 |
| Treibgas (Butan, Isobutan, Propan) | 85,00 | 85,00 | 85,00 | 85,00 |

**Patentansprüche**

1. Aerosolzubereitung umfassend

   a.) ein oder mehrere pulverförmige Stoffe,
   b.) mindestens ein Suspendiermittel, gewählt aus der Gruppe der Schichtsilikate, und
   c.) ein Aktivierungssystem aus Triethylcitrat und Dipropylenglykol im Verhältnis 10:1 bis 1:10.

2. Zubereitung nach Anspruch 1, **dadurch gekennzeichnet, dass** als pulverförmige Stoffe Antitranspirantwirkstoffe gewählt werden.

3. Zubereitung nach Anspruch 1, **dadurch gekennzeichnet, dass** der Zubereitung polare Stoffe, wie Parfumöle, zugesetzt sind.

4. Zubereitung nach Anspruch 1, **dadurch gekennzeichnet, dass** als Schichtsilikate Montmorillonit, Kaolinit, Ilit, Beidellit, Nontronit, Saponit, Hectorit, Bentonit und/oder Smectit, insbesondere Hectorit, gewählt werden.

5. Zubereitung nach einem der vorstehenden Ansprüche, **dadurch gekennzeichnet, dass** das Verhältnis Schichtsilikat zu Aktivierungssystem 15:1 bis 1:1, vorzugsweise 10:1 bis 2:1, beträgt.

6. Verwendung eines Aktivierungssystems aus Triethylcitrat und Dipropylenglykol im Verhältnis 10:1 bis 1:10 zur Überführung von Schichtsilikaten in eine quellbare Form.

7. Verwendung eines Aktivierungssystems aus Triethylcitrat und Dipropylenglykol im Verhältnis 10:1 bis 1:10 zur Reduktion der Absetzgeschwindigkeit von pulverförmigen Stoffen in einer Aerosolzubereitung umfassend ein oder mehrere pulverförmige Stoffe, mindestens ein Suspendiermittel, gewählt aus der Gruppe der Schichtsilikate.

8. Verwendung nach Anspruch 7, **dadurch gekennzeichnet, dass** als pulverförmige Stoffe Antitranspirantwirkstoffe gewählt werden.

9. Verwendung nach Anspruch 7 oder 8, **dadurch gekennzeichnet, dass** der Zubereitung polare Stoffe, insbesondere Parfumöle, zugesetzt sind.

**Claims**

1. Aerosol preparation comprising

   a) one or more pulverulent substances,
   b) at least one suspension agent selected from the group of sheet silicates, and
   c) an activation system composed of triethyl citrate and dipropylene glycol in the ratio from 10:1 to 1:10.

2. Preparation according to Claim 1, **characterized in that** the pulverulent substances selected are antiperspirant active ingredients.

3. Preparation according to Claim 1, **characterized in that** polar substances such as perfume oils are added to the preparation.

4. Preparation according to Claim 1, **characterized in that** the sheet silicates selected are montmorillonite, kaolinite, illite, beidellite, nontronite, saponite, hectorite, bentonite and/or smectite, especially hectorite.

5. Preparation according to any of the preceding claims, **characterized in that** the ratio of sheet silicate to activation system is from 15:1 to 1:1, preferably from 10:1 to 2:1.

6. Use of an activation system composed of triethyl citrate and dipropylene glycol in the ratio from 10:1 to 1:10 for converting sheet silicates into a swellable form.

7. Use of an activation system composed of triethyl citrate and dipropylene glycol in the ratio from 10:1 to 1:10 for reducing the settling rate of pulverulent substances in an aerosol preparation comprising one or more pulverulent substances and at least one suspension agent selected from the group of sheet silicates.

8. Use according to Claim 7, **characterized in that** the pulverulent substances selected are antiperspirant active ingredients.

9. Use according to Claim 7 or 8, **characterized in that** polar substances, especially perfume oils, are added to the preparation.

**Revendications**

1. Préparation d'aérosol, comprenant :

   a.) une ou plusieurs substances en poudre,
   b.) au moins un agent de suspension, choisi dans le groupe des phyllosilicates, et
   c.) un système d'activation constitué par du citrate de triéthyle et du dipropylène glycol en un rapport de 10:1 à 1:10.

2. Préparation selon la revendication 1, **caractérisée en ce que** des agents actifs antitranspirants sont choisis en tant que substances en poudre.

3. Préparation selon la revendication 1, **caractérisée en ce que** des substances polaires, telles que des huiles parfumées, sont ajoutées à la préparation.

4. Préparation selon la revendication 1, **caractérisée en ce que** de la montmorillonite, de la kaolinite, de l'illite, de la beidellite, de la nontronite, de la saponite, de l'hectorite, de la bentonite et/ou de la smectite, notamment de l'hectorite, sont choisies en tant que phyllosilicates.

5. Préparation selon l'une quelconque des revendications précédentes, **caractérisée en ce que** le rapport entre le phyllosilicate et le système d'activation est de 15:1 à 1:1, de préférence de 10:1 à 2:1.

6. Utilisation d'un système d'activation constitué par du citrate de triéthyle et du dipropylène glycol en un rapport de 10:1 à 1:10 pour la transformation de phyllosilicates en une forme gonflable.

7. Utilisation d'un système d'activation constitué par du citrate de triéthyle et du dipropylène glycol en un rapport de 10:1 à 1:10 pour la réduction de la vitesse de décantation de substances en poudre dans une préparation d'aérosol comprenant une ou plusieurs substances en poudre, au moins un agent de suspension choisi dans le groupe des phyllosilicates.

8. Utilisation selon la revendication 7, **caractérisée en ce que** des agents actifs antitranspirants sont choisis en tant que substances en poudre.

9. Utilisation selon la revendication 7 ou 8, **caractérisée en ce que** des substances polaires, notamment des huiles parfumées, sont ajoutées à la préparation.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2442314 A1
- DE 2608123
- DE 3015450 A1
- DE 102008022434
- DE 102007031452
- DE 10237054
- EP 2014273 A1
- EP 2090284 A1
- FR 2859907 A1
- WO 2010112460 A2
- DE 202006015778 U1
- WO 2007141289 A1
- DE 4009347